# EUROPEAN PATENT SPECIFICATION

(11) **EP 1 667 987 B1**
(45) Date of publication and mention of the grant of the patent: **23.07.2008**
(21) Application number: 04765061.9
(22) Date of filing: 10.09.2004
(51) Int. Cl.: C07D 307/88

(54) **PROCESS FOR THE PRODUCTION OF MYCOPHENOLATE MOFETIL**
VERFAHREN ZUR HERSTELLUNG VON MYCOPHENOLATMOFETIL
PROCEDE DE PRODUCTION DE MYCOPHENOLATE MOFETIL

(30) Priority: 11.09.2003 AT 14332003; 17.12.2003 AT 20292003; 17.12.2003 AT 20302003
(43) Date of publication of application: 14.06.2006
(73) Proprietor: SANDOZ AG, 4056 Basel (CH)
(72) Inventor: GREIL, Julia, A-6233 Kramsach (AT); LUDESCHER, Johannes, A-6252 Breitenbach (AT); WOLF, Siegfried, A-6230 Brixlegg (AT)
(74) Representative: Kröger, Bernd
(86) International application number: PCT/EP2004/010134
(87) International publication number: WO 2005/023791

(56) References cited:
- EP-A- 0 281 713
- WO-A-00/34503
- WO-A-95/07902
- WO-A-02/100855
- US-A- 4 686 234
- US-A- 4 727 069
- US-A- 4 748 173
- US-A- 5 247 083

## Description

The present invention relates to new processes in the purification of mycophenolate mofetil of formula I

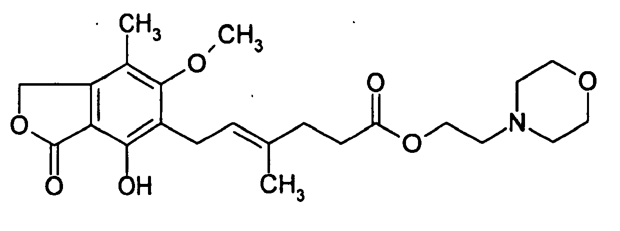

As a result of its immunosuppressive, antiinflammatory, antiviral and anti-tumour activity, mycophenolate mofetil [mycophenolic acid 2-(4-morpholinyl)ethyl ester, or (4E)-6-(1,3-dihydro-4-hydroxy-6-methoxy-7-methyl-3-oxo-5-isobenzofuranyl)-4-methyl-4-hexenoic acid-2-(4-morpholinyl)-ethyl ester according to Merck Index / 13^{th} Edition / Monograph number 6352] is used as the active component of pharmaceutical preparations, e.g. in the prevention of rejection reactions following transplantations, in the treatment of autoimmune diseases, psoriasis, inflammatory disorders such as rheumatic arthritis, as well as viral illnesses and tumours.

The production of mycophenolate mofetil from the fermentation product of mycophenolic acid and 4-(2-hydroxyethyl)morpholine is a demanding procedure because of the basic function of the morpholine moiety and the polyfunctionality of the mycophenolic acid.

One known process comprises, for example, esterification without a catalyst with relatively long reaction times.

Other known processes for the production of mycophenolate mofetil are carried out, for example, via an acid chloride of mycophenolic acid which is produced with thionyl chloride, or via activation with carbodiimide. These two methods are regarded as having little suitability or none at all for producing mycophenolate mofetil in pharmaceutically acceptable purity, especially because of impurities that arise, i.e. by-products occurring during the process. These by-products are, in particular, dimers, for example of formula II:

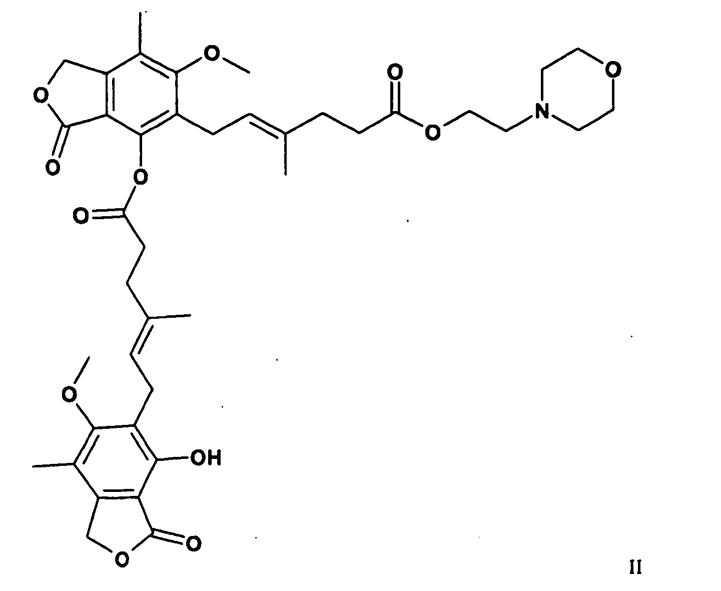

It has now surprisingly been found that mycophenolate mofetil can be produced in high purity and in a high yield by modifying the above-described acid chloride process. This new process for the production of mycophenolate mofetil, as described here, is thereby extremely attractive in consideration of economic yield.

It has also surprisingly been found that the dimeric by-products, hereinafter called "dimers", for example of formula II, may be removed specifically from solutions or suspensions containing mycophenolate mofetil and the above-mentioned dimeric by-products, by means of treatment with a primary or secondary amine so that mycophenolate mofetil can subsequently be isolated therefrom in a good yield and in high purity, i.e. practically free from dimers.

This new process for purifying mycophenolate mofetil, described here, is thus of interest in industrial use.

Using the process according to the invention, mycophenolate mofetil is obtained as the free base in a very high degree of pharmaceutically acceptable purity.

An aspect of the invention comprises a process for the purification of mycophenolate mofetil of formula I by removing its by-products, in particular its dimeric by-products, whereby a solution or a suspension containing mycophenolate mofetil and its by-products, in particular its dimeric by-products, is treated with a primary or secondary amine.

Treatment with a primary or secondary amine is hereby effected in such a way that the solution or suspension of mycophenolate mofetil and its by-products is brought into contact with the amine under controlled conditions.

The process according to the invention for the purification of mycophenolate mofetil by treatment with the amine is especially suitable for extracting mycophenolate mofetil from reaction mixtures as they are obtained upon esterification of a reactive derivative of mycophenolic acid with 4-(2-hydroxyethyl)morpholine.

A further aspect of the invention comprises a process for the purification of mycophenolate mofetil, which consists of the following reaction steps:
a) activation of mycophenolic acid by forming a reactive derivative in an inert solvent according to known methods,
b) reacting the reactive derivative of mycophenolic acid with 4-(2-hydroxyethyl)morpholine by esterifying to mycophenolate mofetil under acidic reaction conditions,
c) treating it with a primary or secondary amine, and
d) isolating the free base of mycophenolate mofetil according to known methods.

Another aspect of the invention comprises a process for the purification of mycophenolate mofetil which contains by-products, which comprises the following reaction steps:
a) preparing a solution or suspension of mycophenolate mofetil as a free base in an inert solvent,
b) treating it with a primary or secondary amine, and
c) isolating the mycophenolate mofetil.

A further aspect of the invention comprises a process for the production and purification of mycophenolate mofetil, which comprises the following steps:
a) activation of mycophenolic acid by forming a reactive derivative by known methods,
b) reacting the reactive derivative of mycophenolic acid with 4-(2-hydroxyethyl)morpholine by esterifying to mycophenolate mofetil under acidic reaction conditions,
c) treating the reaction mixture with a primary or secondary amine,
d) isolating mycophenolate mofetil through the formation of an acid addition salt, and
e) releasing the free base of mycophenolate mofetil from the acid addition salt by known methods.

The acid addition salt of mycophenolate mofetil is, for example, the oxalate.

Through treatment with the amine according to the invention, mycophenolate mofetil can be obtained as the free base in a very high degree of pharmaceutically acceptable purity, whereby the content of dimers lies at or below the detectability threshold, i.e. with a dimer content of 0.1 to 0.03% (area percent HPLC) or less.

The mycophenolate mofetil, which has been purified by the process according to the invention, and which is free from or has a low content of dimers, for example the free base of the mycophenolate mofetil, may be used to produce pharmaceutically acceptable salts of mycophenolate mofetil by known methods.

By an "inert solvent" as described in the present invention is understood a solvent which is inert to the reaction partners under the reaction conditions described here, especially a solvent that is immiscible or only very poorly miscible with water, such as acetic acid (C₁-C₄) alkylester, for example ethyl acetate or isopropyl acetate, or halogenated hydrocarbons, for example dichloromethane, optionally in the presence of a cosolvent. Suitable cosolvents are amides, such as N,N-dimethylformamide or N,N-dimethylacetamide, or cyclic amides, for example N-methylpyrrolidone, or cyclic ureas, for example 1,3-dimethyl-2-imidazolidinone (DMEU) or 1,3-dimethyl-3,4,5,6-tetrahydro-2(1H)-pyrimidinone (DMPU).
Expecially preferred combinations are mixtures of ethyl acetate and N,N-dimethylformamide, and of dichloromethane and N,N-dimethylformamide.

By "pharmaceutically acceptable salts"of mycophenolate mofetil, as described in the present invention, is understood for example the hydrochloride.

By "dimeric by-products" or "dimers", as described here, are understood dimeric compounds which are formed during the esterification reaction of the reactive derivative of mycophenolic acid with 4-(2-hydroxyethyl)morpholine to form mycophenolate mofetil. Apart from the dimers, other by-products may also be formed, for example trimeric or polymeric by-products.

By "reactive derivative of mycophenolic acid" as described here, is understood for example an acid halide, preferably an acid chloride.

Detection of the dimeric content of mycophenolate mofetil is carried out using known HPLC methods; the detectability threshold lies at 0.03% (area percent HPLC), i.e. dimeric contents of < 0.03% do not count as being detectable.

Suitable primary or secondary amines in current organic solvents are soluble amines of formula I 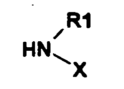
- whereby R₁ may represent hydrogen or Y, and
- whereby X and Y may be identical or different, and X or Y may each be
   a) hydrogen, or
   b) an optionally substituted C₁-C₁₂-alkyl group, which is optionally interrupted by a hetero atom from the series nitrogen, oxygen or sulphur or by an alkylene group, or
   c) an optionally substituted aryl group, or
   d) an optionally basic aromatic heterocycle, or
   e) an optionally substituted saturated or unsaturated aliphatic 3- to 8-membered ring, which may optionally contain hetero atoms from the series nitrogen or oxygen, or
whereby X with R1 may form an optionally substituted saturated or unsaturated aliphatic 3- to 8-membered ring, which may optionally contain hetero atoms from the series nitrogen or oxygen.

Suitable substituents are alkyl, carboxyl, alkoxy or hydroxy groups, or aryl groups which optionally contain alkyl, carboxyl, alkoxy or hydroxy groups, or amino groups, monoalkyl- or monoaryl-amines, dialkyl- or diaryl-amines, a trialkylammonium or triarylammonium group, a cyclic amine or a basic heterocycle.

Suitable amines are C₂-C₆-monoalkylamines, for example n-butylamine, and di- or polyamines, for example ethylenediamine, diaminobutane, diaminopentane, diaminohexane, diaminocyclohexanes, or dimethylaminopropylamine, for example 3-N,N-dimethylamino-1-propylamine, which following the reaction with the dimers, produce an amide derivative of mycophenolic acid which is readily extractable from organic solvents.

By "current organic solvents", as described above, are understood ketones, for example acetone or methyl isobutyl ketone, C₁-C₄-alcohols, nitriles, for example acetonitrile, or the above-defined inert solvents, optionally in the presence of the above-mentioned cosolvents, or mixtures thereof.

The production of mycophenolate mofetil may be carried out, for example, as follows:
The activation of mycophenolic acid may take place for example by Vilsmeier technology (according to Vilsmeier A., Chem.-Ztg. [Chem Journal] 75, 133-135, 1951; CD Römpp Chemie Lexikon - Version 1.0, Stuttgart/New York: Georg Thieme Verlag 1995), for example with an isolated Vilsmeier reagent, for example N,N-dimethyl(chloromethylene)iminium chloride, or by *in situ* activation, for example with an organic amide such as N,N-dimethylformamide or N,N-dimethylacetamide in combination with a halogenation agent, for example oxalyl chloride.

The amount of activation reagent can be kept small for economic reasons. For example, 1 to 1.5 molar equivalents, preferably 1.05 to 1.3 molar equivalents, of Vilsmeier reagent, or Vilsmeier reagent prepared *in situ,* are used. The *in situ* preparation of Vilsmeier reagent in the combination N,N-dimethylformamide and oxalyl chloride is preferred, whereby the ratio of N,N-dimethylformamide to oxalyl chloride is 10 : 1 to 1 : 2, preferably 1 : 1 to 1 : 1.2.

Suitable solvents for activation of the mycophenolic acid are the above-described inert solvents, optionally in the presence of a cosolvent as listed above.

The activation of mycophenolic acid may be carried out at temperatures of -20°C to room temperature, for example from -20°C to +25°C, for example from -10°C to +10°C, preferably at 0°C.

Alternatively, the activation of mycophenolic acid may also take place with current halogenation agents, for example thionyl chloride, or halogenated phosphorus compounds, for example phosphorus trichloride, phosphorus oxychloride and phosphorus pentachloride, or 2,4,6-trichloro-1,3,5-triazine or 5-chloro-4,6-dimethoxy-1,3,5-triazine.

The subsequent reaction of the reactive derivative of mycophenolic acid, for example the acid chloride, with 4-(2-hydroxyethyl)morpholine is carried out for example in such manner that the alcohol 4-(2-hydroxyethyl)morpholine is added to the solution of the acid chloride under controlled conditions, for example over ca. 2 to ca. 180 minutes, preferably over ca. 10 to ca. 60 minutes, so that the excess of free alcohol is kept at a relatively low level.

The hydrochloric acid being released in the acylation reaction thereby lowers the basicity of the reaction mixture, so that under the resultant acidic reaction conditions the formation of by-products is greatly suppressed. The reaction progresses substantially quantitatively with little excess of 4-(2-hydroxyethyl)-morpholine.

For the esterification reaction described here, for example 0.8 to 2.5 molar equivalents, preferably 1.05 to 1.3 molar equivalents, of 4-(2-hydroxyethyl)-morpholine are added.

According to a preferred variant of the process described here, the esterification reaction is started at lower temperatures and is carried out at higher temperatures in order to complete the reaction.

In a further preferred variant, 4-(2-hydroxyethyl)morpholine is added at temperatures of 0°C to 40°C, for example from 10°C to 30°C, preferably at temperatures around 20°C, and the reaction is brought to an end at temperatures of 40°C to 120°C, preferably at 40°C to 60°C.

The reaction times for the esterification reaction according to the invention are, for example, ca. 3 to ca. 20 hours, preferably ca. 5 to ca. 15 hours.

According to one variant of the process, the mycophenolate mofetil may subsequently be isolated from the acidic reaction mixture by forming an acid addition salt, for example the oxalate or hydrochloride, and subsequently releasing the free base.

If the above-described esterification reaction is carried out in a corresponding apolar solvent, for example an acetic acid (C₁-C₄)-alkylester, for example ethyl acetate or isopropyl acetate, mycophenolate mofetil precipitates from the acidic reaction mixture directly as the hydrochloride.
The mycophenolate mofetil hydrochloride is isolated by known methods, for example by filtration.

Release of the free base may be carried out for example in a two-phase system - water with an organic solvent - with the assistance of a base such as sodium or potassium hydroxide, an alkali carbonate or alkali hydrogen carbonate, and mycophenolate mofetil is crystallised for example from ethyl acetate or isopropyl acetate, or from an alcohol such as ethanol or isopropanol or a ketone such as acetone or a combination of said solvents, optionally after evaporation of the solvent.
The preferred organic solvent for release of the free base is ethyl acetate or isopropyl acetate.

After the esterification reaction has taken place as described above, the reaction mixture is neutralised, i.e. brought to a pH value of 5 - 10, preferably 7 - 9.5, preferably 6 - 8, for example by treating the reaction mixture with water or with diluted aqueous base, for example sodium or potassium hydroxide, or an alkali carbonate or alkali hydrogen carbonate.

In another aspect of the invention, subsequently to the above-described neutralisation, the reaction mixture is treated with the primary or secondary amine as follows:
The amine is added to the reaction mixture either as a liquid or as a solution in one of the inert solvents as described above.
   The amine then reacts with the dimers present in the reaction mixture by forming amides of mycophenolic acid, which are separated in the subsequent isolation of the free base of mycophenolate mofetil..

Without committing themselves to a definite mechanism, the present applicants assume that the amine hereby selectively cleaves the phenolic ester of the dimeric by-products, whereupon degradation of these dimers takes place.

In a similar manner, tri- or polymeric by-products which may also be present, are cleaved and broken down by the amine.

The amount of amine used is not critical. Usually, for the above-described process, 0.05 to 0.6 molar equivalents, preferably 0.1 to 0.5 molar equivalents, are used based on the mycophenolic acid employed.

The temperature during treatment of the reaction mixture with the amine depends on the solvent or solvent mixture employed, and is normally in a range of -20°C to +50°C, for example 0°C to 40°C, preferably at room temperature or temperatures up to 40°C.

The time for the treatment of the reaction mixture with the primary or secondary amine is, for example, a few minutes to a few hours, and is dependent on the amount of amine used.

The pressure prevailing during treatment is not critical, i.e. it may correspond to atmospheric pressure, but may also be lower or higher than this.

When the reaction of the amine with the dimers has ended, the excess amine is extracted off using an acid by known methods, for example with aqueous hydrochloric acid. Subsequently, the reaction mixture is neutralised, i.e. brought to a pH value of 5 - 9, preferably 6 - 8 for example with a base, for example sodium or potassium hydroxide, or an alkali carbonate or alkali hydrogen carbonate. Mycophenolate mofetil as a free base may then be isolated by known methods, for example from ethyl acetate or isopropyl acetate.

In another embodiment of the process, mycophenolate mofetil may be isolated from a reaction mixture as the free base as it is produced, for example by using one of the above-described halogenated hydrocarbons, preferably dichloromethane, optionally in combination with a cosolvent, whereby when the esterification reaction has ended, the reaction mixture is neutralised as described.
The reaction mixture is then treated with the amine and the acidic extraction and further neutralisation are carried out as described above. Subsequently, most of the halogenated hydrocarbon, preferably the dichloromethane, is removed by evaporation and then mycophenolate mofetil is isolated as the free base from ethyl acetate or isopropyl acetate by known methods.

In another embodiment of the process, the reaction mixture which is produced preferably by using one of the above-described halogenated hydrocarbons, preferably dichloromethane, optionally in combination with a cosolvent, is neutralised as described above after the esterification reaction has ended. The halogenated hydrocarbon, preferably dichloromethane, is then evaporated, ethyl acetate is added to the resulting residue; and the reaction mixture is treated with the amine, extracted under acidic conditions, and neutralised again as described above. Mycophenolate mofetil is subsequently isolated as the free base from ethyl acetate or isopropyl acetate according to known methods.

Another aspect of the invention comprises a process for the purification of mycophenolate mofetil which contains by-products, which can be carried out as follows:
Mycophenolate mofetil which contains, for example, dimeric by-products, for example mycophenolate mofetil as the free base or mycophenolate mofetil as the acid addition salt, for example as the oxalate or hydrochloride, is dissolved or suspended in a solvent which is immiscible with or poorly miscible with water, for example an acetic acid-C₁-C₄-alkylester, or for example a halogenated hydrocarbon such as dichloromethane, optionally in the presence of a cosolvent, for example an organic amide or an alcohol.
Subsequently, where the mycophenolate mofetil contains as dimeric by-products mycophenolate mofetil as the acid addition salt, first of all the free base of mycophenolate mofetil is released.
   Release of the free base may take place, for example, in a two-phase system - water with an organic solvent - with the assistance of a base such as sodium or potassium hydroxide, an alkali carbonate or alkali hydrogen carbonate.

The resulting solution or suspension is the treated with the corresponding primary or secondary amine as described above, breaking down the dimeric by-products. Normally 0.05 to 0.6 molar equivalents of amine, preferably 0.1 to 0.5 molar equivalents, are used for this based on the mycophenolate mofetil employed.

Afterwards, mycophenolate mofetil is isolated by known methods, for example crystallised from ethyl acetate or an alcohol such as ethanol or isopropanol, or a ketone such as acetone, or a combination of the said solvents, optionally after removal of the solvent by evaporation. The preferred organic solvent is ethyl acetate or isopropyl acetate.

Alternatively, mycophenolate mofetil, for example as the free base, is dissolved or suspended in a solvent which is miscible with water, for example acetone, or an alcohol such as methanol, the resulting solution or suspension is treated with the corresponding amine, and then mycophenolate mofetil is extracted in a solvent which is immiscible with or poorly miscible with water, and mycophenolate mofetil is isolated by known methods.

An additional advantage of the process described here is the relatively short reaction times during the esterification reaction.

By employing the process, described as another aspect of the invention, for the purification of mycophenolate mofetil through treatment with an amine, mycophenolate mofetil is obtained for example as the free base or as the oxalate with a content of dimers of a maximum of 0.15% (area percent HPLC) or less, for example 0.1 to 0.03% (area percent HPLC) or less, for example 0.08% or less, for example 0.05% or less.

The process described here for the production of mycophenolate mofetil is therefore economically attractive when producing mycophenolate mofetil as a free base or as the pharmaceutically acceptable salts thereof, for example the hydrochloride, in pharmaceutically acceptable pure form.

Furthermore, the treatment with the amine described here is of interest in industrial application, since it enables high yields to be achieved in relatively short reaction times.

The following examples are intended to illustrate the invention more fully, without limiting its scope. All temperatures are given in degrees celsius and are uncorrected.

### Example 1:

### Production of mycophenolate mofetil through the formation of the mycophenolate mofetil hydrochloride

### Example 1a:

### Production of mycophenolate mofetil hydrochloride

9.094 g of mycophenolic acid are added at room temperature to a mixture of 140 ml of ethyl acetate and 3.12 ml of N,N-dimethylformamide. The suspension is cooled to 0°, and subsequently 3.05 ml of oxalyl chloride, dissolved in 15 ml of ethyl acetate, are added dropwise over ca. 20 minutes, whereby a solution is obtained. The solution is stirred for a further 140 minutes at 0° to 2° and then heated to 20°. Then, a solution of 4.16 ml of 4-(2-hydroxyethyl)morpholine in 20 ml of ethyl acetate is added over the course of ca. 20 minutes at 20° to 22°, whereby a suspension forms already after the first drops. The suspension is heated to 60° and is stirred at this temperature for ca. 20 hours. The title compound is then isolated by filtering the hot suspension through a suction filter, washed twice, each time with 25 ml of ethyl acetate heated to 60°, and dried in a vacuum over night at room temperature.
Weight of mycophenolate mofetil hydrochloride: 11.85 g
HPLC purity: 97.8%

### Example 1b:

### Production of mycophenolate mofetil as the free base

10 g of the mycophenolate mofetil hydrochloride obtained in example 1a are suspended in 100 ml of ethyl acetate. 50 ml of water are added and stirred until a two-phase solution is obtained. Then, the pH value is adjusted from ca. 2.0 to pH 7.4 to 7.5 using ca. 30 ml of saturated NaHCO₃ solution, the phases are separated and the organic phase is washed first of all with a mixture of 50 ml of water and 2.5 ml of saturated NaHCO₃ solution, and then twice with 50 ml of water each time. The ethyl acetate phase is then mixed with 1 g of activated carbon and stirred for 10 minutes at room temperature. The activated carbon is removed by filtration, the filter cake washed with 10 ml of ethyl acetate, and the combined phases are concentrated to ca. 40 g by evaporation in a vacuum. The residue is seeded with the title compound, the resulting suspension is stirred for one hour at room temperature and then cooled to ca. -20°. After standing over night in a refrigerator at ca. -20°, the title compound is isolated through a suction filter, washed with 5 ml of cold ethyl acetate and dried over night in a vacuum.
Weight of mycophenolate mofetil as the free base: 6.71 g
HPLC purity: 99.5%

### Example 2:

### Purification of mycophenolate mofetil by means of treatment with n-butylamine

13.64 g of mycophenolic acid are dissolved at room temperature in a mixture of 196.6 ml of dichloromethane and 3.54 ml of N,N-dimethylformamide. The solution is cooled to ca. 0°, and a solution of 3.85 ml of oxalyl chloride in 14 ml of dichloromethane is added dropwise to the thin suspension through a dropping funnel over the course of ca. 35 minutes, whereby a clear solution is again obtained. Afterwards, the dropping funnel is rinsed with 10 ml of dichloromethane. After the addition is complete, the mixture is stirred for about a further 120 minutes at 0°. The solution is then brought to ca. 20°. A solution of 6.25 ml of 4-(2-hydroxyethyl)morpholine in 30 ml of dichloromethane is added dropwise over the course of ca. 25 minutes. The dropping funnel is rinsed with 10 ml of dichloromethane and the solution is subsequently boiled under reflux for ca. 12 hours. Afterwards, the solution is cooled to 20° and mixed with 127 ml of water. The two-phase solution is stirred for ca. 30 minutes at 15° to 20°, and then the pH value is adjusted to ca. 8.0 with ca. 200 ml of saturated NaHCO₃ solution. The phases are separated and the aqueous phase is then extracted with 50 ml of dichloromethane. The combined organic phases are mixed with 75 ml of water and 10 ml of saturated NaHCO₃ solution, the mixture is stirred for ca. 20 minutes and the phases are separated. 1.75 ml of n-butylamine are added and the solution is stirred for ca. 4 hours. The dichloromethane phase is then extracted with a mixture of 50 ml of water and 10 ml of 2N HCl, the phases are separated, the organic phase is washed with 50 ml of water and 25 ml of saturated NaHCO₃ solution, and subsequently with 50 ml of water. The solution is mixed with 1.5 g of activated carbon, the mixture stirred for 10 minutes, and the activated carbon filtered off through a suction filter. The filter cake is washed with 10 ml of dichloromethane and mixed with the filtrate.
The solvent is evaporated in a vacuum in a rotary evaporator and the solid residue is brought to solution at 50° with 65 ml of ethyl acetate. The solution is cooled to ca. 20°, seed crystals of the title compound are added, and the suspension is stirred first of all for one hour at ca. 20°, then for one hour with ice cooling and for a further two hours at ca. -20°. The suspension is then left to stand over night at ca. -20°, the product is isolated through a suction filter, washed with 10 ml of ethyl acetate of -20°, and dried at room temperature over night in a vacuum.
Weight of mycophenolate mofetil as the free base: 15.84 g
Dimer content: undetectable, i.e. < 0.03% (area percent HPLC)

### Example 3:

### Reference example: Production of mycophenolate mofetil without the treatment with an amine

13.64 g of mycophenolic acid are dissolved at room temperature in a mixture of 196.6 ml of dichloromethane and 3.54 ml of N,N-dimethylformamide. The solution is cooled to ca. 0°, and a solution of 3.85 ml of oxalyl chloride in 14 ml of dichloromethane is added dropwise to the thin suspension through a dropping funnel over the course of ca. 35 minutes, whereby a clear solution is again obtained. Afterwards, the dropping funnel is rinsed with 10 ml of dichloromethane. After the addition is complete, the mixture is stirred for about a further 120 minutes at 0°. The solution is then brought to ca. 20°. A solution of 6.25 ml of 4-(2-hydroxyethyl)morpholine in 30 ml of dichloromethane is added dropwise over the course of ca. 25 minutes. The dropping funnel is rinsed with 10 ml of dichloromethane and the solution is subsequently boiled under reflux for ca. 12 hours. Afterwards, the solution is cooled to 20° and mixed with 127 ml of water. The two-phase solution is stirred for ca. 30 minutes at 15° to 20°, and then the pH value is adjusted to ca. 8.0 with ca. 200 ml of saturated NaHCO₃ solution. The phases are separated and the aqueous phase is then extracted with 50 ml of dichloromethane. The combined organic phases are mixed with 75 ml of water and 10 ml of saturated NaHCO₃ solution, the mixture is stirred for ca. 20 minutes and the phases are separated. The dichloromethane phase is washed twice, each time with 75 ml of water, and then 1.5 g of activated carbon are added. The mixture is stirred for ca. 10 minutes, the carbon is filtered off and the activated carbon is washed with 15 ml of dichloromethane.
The combined dichloromethane phases are concentrated by evaporation in a vacuum on a rotary evaporator. The residue is dissolved in 65 ml of ethyl acetate at ca. 50° and then cooled to ca. 20°. Seed crystals of the title compound are added, the suspension is stirred first of all for ca. 1 hour at room temperature, then for ca. 1 hour whilst cooling with ice, followed by 2 hours at ca. -20°. The suspension is then left to stand over night at ca. -20° and the product is isolated through a suction filter. The product is washed with 10 ml of ethyl acetate of -20° and subsequently dried in a vacuum at room temperature.
Weight of mycophenolate mofetil as the free base: 15.99 g
Dimer content: 0.17 % (area percent HPLC)

### Example 4:

### Purification of mycophenolate mofetil by means of treatment with 3-N,N-dimethylamino-1-propylamine

To a solution of 1190 ml of dichloromethane and 17.7 ml of N,N-dimethylformamide are added 68.2 g of mycophenolic acid. The solution is cooled to 0°C and 19.6 ml of oxalylchloride are added via dropping funnel during ca. 15 min. The reaction solution is then stirred at 0°C for 2 hours. The mixture is then heated to ca. 22°C and 31.2 ml of 4-(2-hydroxyethyl)morpholine is added dropwise to this solution over the course of ca. 25 min and the mixture is then refluxed for 14 hours while purging N₂ through the reaction mixture. The mixture is then cooled to ca. 22°C and 550 ml of H₂O are added. The pH of the mixture is adjusted to pH 5.4 with ca. 210 ml of 1N NaOH, and the phases are separated and to the organic phase are added 550 ml of H₂O. The pH is adjusted with approximately 10 ml of 1N NaOH to 9.1. The phases are separated, the organic phase is washed with 180 ml of H₂O keeping the pH at 9.1 with a few drops of 1N NaOH. The phases are separated and the organic phase is evaporated in vacuo to an oil. The residue of evaporation is dissolved in 1250 ml of isopropylacetate. The solution is heated to approximately 40°C and 5.3 ml of 3-N,N-dimethylamino-1-propylamine is added. The mixture is stirred at 40°C for ca. 80 minutes. 250 ml of H₂O are added. The pH is adjusted to 6.4 with ca. 41 ml of 2N HCl. The phases are separated and to the organic phase are added 250 ml of H₂O. The pH is adjusted to ca. 8.9 with approximately 1 ml of 1N NaOH, the phases are separated and 7.5 g of charcoal are added to the organic phase. The suspension is stirred for 10 min and then filtered. The filtrate is concentrated in vacuo to ca. 380 g while the product starts to crystallize. The suspension is then cooled to 0°C and gently stirred at this temperature for 16 hours. The product is then isolated by filtration, washed with 50 ml of cold isopropylacetate and dried for 16 hours at 35°C.
Yield mycophenolate mofetil: 85%
Purity: 99.9% (HPLC)

### Example 5:

### Purification of mycophenolate mofetil by means of treatment with 3-N,N-di methylamino-1-propylamine

13.64 g of mycophenolic acid are dissolved at room temperature in a mixture of 196.5 ml of dichloromethane and 3.54 ml of N,N-dimethylformamide. A solution of 3.92 ml of oxalyl chloride in 15 ml of dichloromethane is added to the thin suspension at 0° through a dropping funnel over the course of ca. 15 minutes.
Afterwards, the dropping funnel is rinsed with 10 ml of dichloromethane and the solution is stirred for two hours at 0°. The assay mixture is then heated to ca. 20° and a solution of 6.24 ml of 4-(2-hydroxyethyl)morpholine in 30 ml of dichloromethane is added dropwise over the course of ca. 25 minutes, the dropping funnel is rinsed with 10 ml of dichloromethane, and then the solution is boiled under reflux for ca. 12 hours. The reaction mixture is then cooled to ca. 20°, 127 ml of water are added and the two-phase mixture is stirred for ca. 30 minutes. A further 100 ml of water are added, and the pH value of the mixture is adjusted to 9.2 with 1N NaOH. The two-phase mixture is stirred for 30 minutes, whereby the pH value is held at between 9 and 9.2 with 1N NaOH. The phases are separated, the water phase is washed with 50 ml of dichloromethane, the organic phases are combined and extracted with 75 ml of water, whereby the pH value is again adjusted to 9 to 9.2 with 1N NaOH. As much dichloromethane as possible is distilled off in a vacuum in a rotary evaporator and the residue is dissolved in 250 ml of ethyl acetate. Then, 1.06 ml of 3-N,N-dimethylamino-1-propylamine are added and the solution is stirred at room temperature for ca. 2.5 hours. Then, a mixture of 50 ml of water and 8.52 ml of 2N HCl is added, the mixture is stirred for 10 minutes and the phases are separated. The organic phase is extracted twice, each time with 50 ml of water. The phases are separated, a mixture of 25 ml of water and 25 ml of saturated NaHCO₃ solution is added, stirred for ca. 10 minutes, the phases are separated and the organic phase is washed with 50 ml of water. The organic solution is then concentrated by evaporation to ca. 76 g in a rotary evaporator under vacuum. The mixture is seeded with mycophenolate mofetil as the free base, the resulting suspension is stirred first of all for ca. 30 minutes at room temperature, then for a further 30 minutes whilst cooling with ice, followed by 1 hour at ca. -20°, and then the suspension is left to stand over night at ca. -20° in a refrigerator.
The product is then isolated through a suction filter, washed in 2 portions with 10 ml of ethyl acetate of -20°, and dried for ca. 6 hours at room temperature in a vacuum.
Weight of mycophenolate mofetil as the free base: 16.05 g
Dimer content: undetectable, i.e. < 0.03% (area percent HPLC)

### Example 6:

### Purification of mycophenolate mofetil as the free base by means of treatment with 3-N,N-dimethylamino-1-propylamine

1.4 g of mycophenolate mofetil obtained in example 6 with a dimeric content of 0.17% (area percent HPLC) are dissolved in 28 ml of ethyl acetate. Then, 80 µl of 3-N,N-dimethylamino-1-propylamine are added and the solution is stirred at room temperature for ca. 2.5 hours. Then, a mixture of 5.6 ml of water and 0.65 ml of 2N HCl is added, the mixture is stirred for ca.10 minutes and the phases are separated. The ethyl acetate phase is then extracted twice, each time with 5.6 ml of water, then with a mixture of 2.8 ml of water and 2.8 ml of saturated NaHCO₃ solution and subsequently with 5.6 ml of water. The ethyl acetate phase is then concentrated to ca. 7 g on a rotary evaporator. The residue of evaporation is cooled to room temperature, and stirred at this temperature for ca. 30 minutes. The suspension is stirred for ca. one further hour whilst cooling with ice, and subsequently stored over night at ca. -20°. The product is then isolated through a suction filter, washed with 1.5 ml of ethyl acetate of -20° and dried in a vacuum for ca. 6 hours.
Weight of mycophenolate mofetil as the free base: 1.08 g
Dimer content: undetectable, i.e. < 0.03% (area percent HPLC)

### Example 7:

### Production of mycophenolate mofetil through the formation of the mycophenolate mofetil oxalate incl. purification by treatment with n-butylamine

### Example 7a:

### Production of mycophenolate mofetil oxalate incl. purification by treatment with n-butylamine

13.64 g of mycophenolic acid are dissolved at room temperature in a mixture of 196.6 ml of dichloromethane and 3.54 ml of N,N-dimethylformamide. The solution is cooled to 0°, and a solution of 3.85 ml of oxalyl chloride in 14 ml of dichloromethane is added dropwise to the thin suspension through a dropping funnel over the course of ca. 35 minutes, whereby a clear solution is again obtained. Afterwards, the dropping funnel is rinsed with 10 ml of dichloromethane. After the addition is complete, the mixture is stirred for about a further 120 minutes at 0°. The solution is then brought to ca. 20°. A solution of 6.25 ml of 4-(2-hydroxyethyl)morpholine in 30 ml of dichloromethane is added dropwise over the course of ca. 25 minutes. The dropping funnel is rinsed with 10 ml of dichloromethane and the solution is subsequently boiled under reflux for ca. 12 hours. Afterwards, the solution is cooled to 20° and mixed with 127 ml of water. The two-phase solution is stirred for ca. 30 minutes at 15° to 20°, and then the pH value is adjusted to ca. 8.0 with ca. 200 ml of saturated NaHCO₃ solution. The phases are separated and the aqueous phase is then extracted with 50 ml of dichloromethane. The combined organic phases are mixed with 75 ml of water and 10 ml of saturated NaHCO₃ solution, the mixture is stirred for ca. 20 minutes and the phases are separated. 1.75 ml of n-butylamine are added and the solution is stirred for ca. one hour. The dichloromethane phase is then extracted with a mixture of 50 ml of water and 10 ml of 2N HCl, the phases are separated, the organic phase is washed with 50 ml of water and 25 ml of saturated NaHCO₃ solution, and the organic phase is washed once more with 50 ml of water. The solution is mixed with 1.5 g of activated carbon, the mixture stirred for 10 minutes, and the activated carbon filtered off through a suction filter. The filter cake is washed with 10 ml of dichloromethane and mixed with the filtrate. To this mixture consisting of the filtrate and the washed phase is added a solution of 4.22 g of water-free oxalic acid in 15 ml of methanol, whereby after adding seed crystals the title compound crystallises out. The suspension is left to stand for ca. one hour at room temperature with occasional stirring, and then stirred for about a further 3 hours with ice cooling. The title compound is subsequently isolated through a suction filter, washed twice, each time with 38 ml of dichloromethane, and dried over night at room temperature in a vacuum drying chamber.
Weight of mycophenolate mofetil oxalate: 19.11 g
Dimer content: 0.05% (area percent HPLC)

### Example 7b:

### Production of mycophenolate mofetil as the free base from purified mycophenolate mofetil oxalate

10 g of the compound from example 9a are suspended in a mixture of 50 ml of water and 100 ml of ethyl acetate. The pH value is then adjusted to 7.4 to 7.5 with 10% KHCO₃ solution, whereby a two-phase solution is produced. The mixture is stirred for ca. 5 minutes, and the phases are subsequently separated. The ethyl acetate phase is subsequently washed with a mixture of 50 ml of water and 2 ml of 10% KHCO₃ solution, followed by 2 washes each time with 50 ml of water. The ethyl acetate phase is concentrated in a vacuum on a rotary evaporator to 40 g, seeded with mycophenolate mofetil, and the resulting suspension is stirred first of all for ca. 1 hour at room temperature, then for ca. 1 hour with ice cooling, then for ca. 2 hours at ca. -20°, and is subsequently stored over night in a refrigerator at ca. -20°. The crystals are subsequently isolated through a suction filter, washed with 5 ml of ethyl acetate of -20° and dried in a vacuum drying chamber at room temperature.
Weight of mycophenolate mofetil as the free base: 7.24 g
Dimer content: 0.04 % (area percent HPLC)

### Example 8:

### Production of mycophenolate mofetil from mycophenolate mofetil oxalate incl. purification by treatment with n-butylamine

10 g of the mycophenolate mofetil oxalate obtained in example 9a, with a content of dimers of 0.05% (area percent HPLC) are suspended in a mixture of 50 ml of water and 100 ml of ethyl acetate. The pH value is then adjusted to 7.4 to 7.5 with 10% KHCO₃ solution, whereby a two-phase solution is produced. The mixture is stirred for ca. 5 minutes, and the phases are subsequently separated. The ethyl acetate phase is subsequently washed with a mixture of 50 ml of water and 5 ml of 10% KHCO₃ solution. 0.4 ml of n-butylamine are added to the organic phase, and the solution is stirred for ca. 120 minutes. Then, the organic phase is extracted with a mixture of 25 ml of water and 2 ml of 2N HCl, then the organic phase is extracted with a mixture of 25 ml of water, and the pH value is adjusted to 8.5 with 10% KHCO₃ solution, followed by a wash with 25 ml of water. The ethyl acetate phase is concentrated in a vacuum on a rotary evaporator to 40 g, seeded with mycophenolate mofetil, and the resulting suspension is stirred first of all for ca. 1 hour at room temperature, then for ca. 1 hour with ice cooling, then for about a further 2 hours at ca. -20°, and is subsequently stored over night in a refrigerator at ca. -20°. The crystals are subsequently isolated through a suction filter, washed with 5 ml of ethyl acetate of -20° and dried in a vacuum drying chamber at room temperature.
Weight of mycophenolate mofetil as the free base: 7.31 g
Dimer content: undetectable, i.e. < 0.03% (area percent HPLC)

## Claims

1. Process for the purification of mycophenolate mofetil [mycophenolic acid 2-(4-morpholinyl)-ethyl ester] of formula I 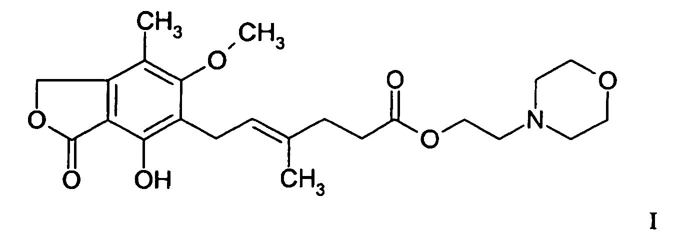 by removing its by-products, whereby a solution or suspension of mycophenolate mofetil is treated with a primary or secondary amine.

2. Process according to claim 1, **characterised in that** the by-products contain dimers.

3. Process according to claim 1 or 2, **characterised in that** the primary or secondary amine has the following formula: 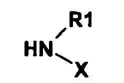
- whereby R₁ is hydrogen or Y, and
- whereby X and Y may be identical or different, and X or Y may each be
a) hydrogen, or
b) an optionally substituted C₁-C₁₂-alkyl group, which is optionally interrupted by a hetero atom from the series nitrogen, oxygen or sulphur or by an alkylene group, or
c) an optionally substituted aryl group, or
d) an optionally basic aromatic heterocycle, or
e) an optionally substituted saturated or unsaturated aliphatic 3- to 8-membered ring, which may optionally contain hetero atoms from the series nitrogen or oxygen, or
- whereby X with R1 forms an optionally substituted saturated or unsaturated aliphatic 3-to 8-membered ring, which may optionally contain hetero atoms from the series nitrogen or oxygen.

4. Process according to claim 3, **characterised in that** the substituents are alkyl, carboxyl, alkoxy or hydroxy groups, or aryl groups which optionally contain alkyl, carboxyl, alkoxy or hydroxy groups, or are amino groups, monoalkyl- or monoaryl-amines, dialkyl- or diaryl-amines, a trialkylammonium or triarylammonium group, a cyclic amine or a basic heterocycle.

5. Process according to claim 4, **characterised in that** the substituents stem from the groups n-butylamine, ethylendiamine, diaminobutane, diaminopentane, diaminohexane, diaminocyclohexane, or dimethylaminopropylamine, for example 3-N,N-dimethylamino-1-propylamine.

6. Process according to one of claims 1 to 5, **characterised in that** the primary or secondary amine is soluble in an organic solvent.

7. Process according to claim 6, **characterised in that** the organic solvent includes a ketone, for example acetone or methyl isobutyl ketone, or a C₁-C₄-alcohol, or a nitrile, for example acetonitrile, or an inert solvent, optionally in the presence of a cosolvent, or mixtures thereof.

8. Process according to claim 7, **characterised in that** the inert solvent is an acetic acid (C₁-C₄)-alkyl ester or a halogenated hydrocarbon, optionally in the presence of a cosolvent.

9. Process according to claims 7 or 8, **characterised in that** the inert solvent is ethyl acetate, isopropyl acetate or dichloromethane, optionally in the presence of a co-solvent.

10. Process according to one of claims 7 to 9, **characterised in that** the cosolvent is an organic amide.

11. Process for the purification of mycophenolate mofetil, **characterised in that** it comprises the following reaction steps:
a) activation of mycophenolic acid by forming a reactive derivative in an inert solvent,
b) reacting the reactive derivative of mycophenolic acid with 4-(2-hydroxyethyl)morpholine by esterifying to mycophenolate mofetil under acidic reaction conditions,
c) treating it with a primary or secondary amine, and
d) isolating the mycophenolate mofetil.

12. Process for the purification of mycophenolate mofetil, which contains by-products, **characterised in that** it comprises the following reaction steps:
a) preparing a solution or suspension of mycophenolate mofetil as a free base in an inert solvent,
b) treating it with a primary or secondary amine, and
c) isolating the mycophenolate mofetil.

13. Process according to claim 12, **characterised in that** the by-products contain dimers.

## Patentansprüche

1. Verfahren zur Reinigung von Mycophenolatmofetil, nämlich Mycophenolsäure-2-(4-morpholinyl)-ethylester der Formel I 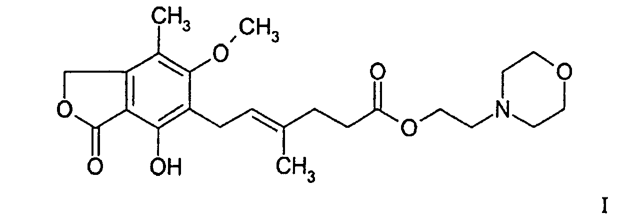 durch Entfernung seiner Nebenprodukte, wodurch eine Lösung oder Suspension von Mycophenolatmofetil mit einem primären oder sekundären Amin behandelt wird.

2. Verfahren nach Anspruch 1, **dadurch gekennzeichnet, dass** die Nebenprodukte Dimere enthalten.

3. Verfahren nach Anspruch 1 oder 2, **dadurch gekennzeichnet, dass** das primäre oder sekundäre Amin die folgende Formel hat 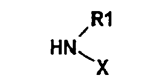
- worin R1 für Wasserstoff oder Y steht, und
- worin X und Y gleich oder verschieden sein können, und X oder Y jeweils stehen können für
a) Wasserstoff oder
b) eine optional substituierte C₁-C₁₂-Alkylgruppe, die optional unterbrochen ist durch ein Heteroatom aus der Reihe Stickstoff, Sauerstoff oder Schwefel, oder durch eine Alkylengruppe, oder
c) eine optional substituierte Arylgruppe, oder
d) einen optional basischen aromatischen Heterocyclus, oder
e) einen optional substituierten gesättigten oder ungesättigten aliphatischen 3- bis 8-gliedrigen Ring, der optional Heteroatome aus der Reihe Stickstoff oder Sauerstoff enthalten kann, oder
- worin X mit R1 optional einen gesättigten oder ungesättigten aliphatischen 3- bis 8-gliedrigen Ring bildet, der optional Heteroatome aus der Reihe Stickstoff oder Sauerstoff enthalten kann.

4. Verfahren nach Anspruch 3, **dadurch gekennzeichnet, dass** die Substituenten Alkyl-, Carboxyl-, Alkoxy-, Hydroxy- oder Arylgruppen sind, die optional Alkyl-, Carboxyl-, Alkoxy- oder Hydroxygruppen enthalten, oder Aminogruppen, Monoalkylamine oder Monoarylamine, Dialkylamine oder Diarylamine, eine Trialkylammoniumgruppe oder Triarylammoniumgruppe, ein cyclisches Amin oder ein basischer Heterocyclus sind.

5. Verfahren nach Anspruch 4, **dadurch gekennzeichnet, dass** die Substituenten aus den Gruppen n-Butylamin, Ethylendiamin, Diaminobutan, Diaminopentan, Diaminohexan, Diaminocyclohexan oder Dimethylaminopropylamino, beispielsweise 3-N,N-Dimethylamino-1-propylamino stammen.

6. Verfahren nach Anspruch 1 bis 5, **dadurch gekennzeichnet, dass** das primäre oder das sekundäre Amin in einem organischen Lösemittel löslich ist.

7. Verfahren nach Anspruch 6, **dadurch gekennzeichnet, dass** das organische Lösemittel ein Keton, beispielsweise Aceton oder Methylisopropylketon, einen C₁-C₄-Alkohol oder ein Nitril umfasst, beispielsweise Acetonitril, oder ein inertes Lösemittel, optional in Gegenwart eines Colösemittels, oder Gemische hiervon.

8. Verfahren nach Anspruch 7, **dadurch gekennzeichnet, dass** das inerte Lösemittel ein Essigsäure-(C₁-C₄)-alkylester oder ein Halogenkohlenwasserstoff ist, optional in Gegenwart eines Colösemittels.

9. Verfahren nach Anspruch 7 oder 8, **dadurch gekennzeichnet, dass** das inerte Lösemittel Ethylacetat, Isopropylacetat oder Dichlormethan ist, optional in Gegenwart eines Colösemittels.

10. Verfahren nach einem der Ansprüche 7 bis 9, **dadurch gekennzeichnet, dass** das Colösemittel ein organisches Amid ist.

11. Verfahren zur Reinigung von Mycophenolatmofetil, **dadurch gekennzeichnet, dass** dieses die folgenden Reaktionsstufen umfasst:
a) Aktivierung von Mycophenolsäure durch Bildung eines reaktiven Derivats in einem inerten Lösemittel,
b) Umsetzung des reaktiven Derivats von Mycophenolsäure mit 4-(2-Hydroxyethyl)-morpholin durch Veresterung zu Mycophenolatmofetil unter sauren Bedingungen,
c) Behandlung dieser Verbindung mit einem primären oder sekundären Amin, und
d) Isolation des Mycophenolatmofetils.

12. Verfahren zur Reinigung von Mycophenolatmofetil, das Nebenprodukte enthält, **dadurch gekennzeichnet, dass** dieses Verfahren die folgenden Reaktionsstufen umfasst:
a) Herstellung einer Lösung oder Suspension von Mycophenolatmofetil als eine freie Base in einem inerten Lösemittel,
b) Behandlung mit einem primären oder sekundären Amin, und
c) Isolation des Mycophenolatmofetils.

13. Verfahren nach Anspruch 12, **dadurch gekennzeichnet, dass** die Nebenprodukte Dimere enthalten.

## Revendications

1. Procédé de purification du mycophénolate mofétil [2-(4-morpholinyl)-éthylester d'acide mycophénolique] de formule 1 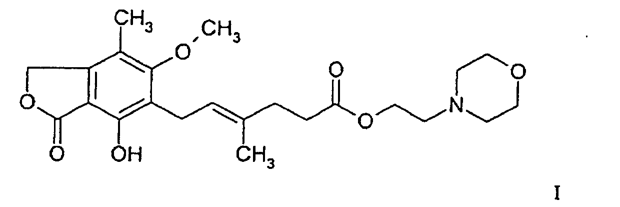 par retrait de ses sous-produits, au moyen d'un traitement d'une solution ou d'une suspension de mycophénolate mofétil par une amine primaire ou secondaire.

2. Procédé selon la revendication 1, **caractérisé en ce que** les sous-produits contiennent des dimères.

3. Procédé selon la revendication 1 ou 2, **caractérisé en ce que** l'amine primaire ou secondaire a la formule suivante : 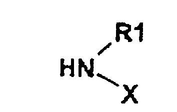
- dans laquelle R₁ est l'hydrogène ou Y, et
- dans laquelle X et Y peuvent être identiques ou différents, et X ou Y peuvent être chacun
a) l'hydrogène, ou
b) un groupe alkyle en C₁-C₁₂ éventuellement substitué, qui est éventuellement interrompu par un hétéroatome de la série de l'azote, de l'oxygène ou du soufre, ou par un groupe alkylène, ou
c) un groupe aryle éventuellement substitué, ou
d) un hétérocycle aromatique éventuellement basique, ou
e) un cycle aliphatique comprenant de 3 à 8 chaînons, saturé ou insaturé, éventuellement substitué, qui peut éventuellement contenir des hétéroatomes de la série de l'azote ou de l'oxygène, ou
- dans laquelle X forme avec R₁ un cycle aliphatique comprenant de 3 à 8 chaînons, saturé ou insaturé, éventuellement substitué, qui peut éventuellement contenir des hétéroatomes de la série de l'azote ou de l'oxygène.

4. Procédé selon la revendication 3, **caractérisé en ce que** les substituants sont des groupes alkyle, carboxyle, alcoxy ou hydroxy, ou des groupes aryle qui contiennent éventuellement des groupes alkyle, carboxyle, alcoxy ou hydroxy, ou sont des groupes amino, des monoalkyl- ou monoaryl-amines, des dialkyl- ou diaryl-amines, un groupe trialkylammonium ou triarylammonium, une amine cyclique ou un hétérocycle basique.

5. Procédé selon la revendication 4, **caractérisé en ce que** les substituants proviennent des groupes n-butylamine, éthylènediamine, diaminobutane, diaminopentane, diaminohexane, diaminocyclohexane, ou diméthylaminopropylamine, par exemple la 3-N,N-diméthylamino-1-propylamine.

6. Procédé selon l'une des revendications 1 à 5, **caractérisé en ce que** l'amine primaire ou secondaire est soluble dans un solvant organique.

7. Procédé selon la revendication 6, **caractérisé en ce que** le solvant organique contient une cétone, par exemple l'acétone ou la méthylisobutylcétone, ou un alcool en C₁-C₄, ou un nitrile, par exemple l'acétonitrile, ou un solvant inerte, éventuellement en présence d'un co-solvant, ou des mélanges de ceux-ci.

8. Procédé selon la revendication 7, **caractérisé en ce que** le solvant inerte est un alkyl(en C₁-C₄)-ester d'acide acétique ou un hydrocarbure halogéné, éventuellement en présence d'un co-solvant.

9. Procédé selon les revendications 7 ou 8, **caractérisé en ce que** le solvant inerte est l'acétate d'éthyle, l'acétate d'isopropyle ou le dichlorométhane, éventuellement en présence d'un co-solvant.

10. Procédé selon l'une des revendications 7 à 9, **caractérisé en ce que** le co-solvant est un amide organique.

11. Procédé de purification du mycophénolate mofétil, **caractérisé en ce qu'**il comprend les étapes de réaction suivantes :
a) activation de l'acide mycophénolique par formation d'un dérivé réactif dans un solvant inerte,
b) réaction du dérivé réactif d'acide mycophénolique avec de la 4-(2-hydroxyéthyl)morpholine par estérification en mycophénolate mofétil dans des conditions de réaction acides,
c) traitement de celui-ci avec une amine primaire ou secondaire, et
d) isolation du mycophénolate mofétil.

12. Procédé de purification du mycophénolate mofétil, qui contient des sous-produits, **caractérisé en ce qu'**il comprend les étapes de réaction suivantes :
a) préparation d'une solution ou d'une suspension de mycophénolate mofétil en tant que base libre dans un solvant inerte,
b) traitement de celle-ci avec une amine primaire ou secondaire, et
c) isolation du mycophénolate mofétil.

13. Procédé selon la revendication 12, **caractérisé en ce que** les sous-produits contiennent des dimères.
